# EUROPÄISCHE PATENTSCHRIFT

(11) **EP 2 456 880 B1**
(45) Veröffentlichungstag und Bekanntmachung des Hinweises auf die Patenterteilung: **14.05.2014**
(21) Anmeldenummer: 10747157.5
(22) Anmeldetag: 20.07.2010
(51) Int. Cl.: C12P 23/00, C12C 11/00

(54) **FERMENTATIONSHILFSMITTEL AUF DER BASIS VON HOPFEN SOWIE FERMENTATIONSVERFAHREN**
FERMENTATION ADDITIVE BASED ON HOPS AND FERMENTATION PROCESS
ADJUVANT DE FERMENTATION À BASE DE HOUBLON ET PROCÉDÉ DE FERMENTATION

(30) Priorität: 22.07.2009 DE 102009034465
(43) Veröffentlichungstag der Anmeldung: 30.05.2012
(73) Patentinhaber: Beta Tec Hopfenprodukte GmbH, 90482 Nürnberg (DE)
(72) Erfinder: BACZYNSKI, Lilith, 91564 Neuendettelsau (DE); BEDDIE, David, Herefordshire HR6 OBG (GB); SMALLMAN, Matthew, Hallow Worcester WR2 6PH (GB)
(74) Vertreter: Stippl, Hubert
(86) Internationale Anmeldenummer: PCT/EP2010/004423
(87) Internationale Veröffentlichungsnummer: WO 2011/009588

(56) Entgegenhaltungen:
- WO-A1-00/53814
- WO-A1-2007/096673
- WO-A2-2004/072291

## Beschreibung

### Anwendungsgebiet der Erfindung

Die vorliegende Erfindung betrifft den Bereich der industriellen Fermentation, wie etwa die Biomasse-Produktion, Enzymproduktion, die Produktion von primären und sekundären mikrobiellen, d.h. durch Mikroorganismen verursachte Metaboliten einschließlich Nahrungsmittelfermentationen.

### Stand der Technik

Die Fermentation betrifft viele wichtige Anwendungen in der Industrie. Obwohl der Begriff "Fermentation" auch engere Bedeutungen haben kann, bedeutet der Begriff im Zusammenhang mit "industrieller Fermentation" generell die Umsetzung von organischen Substanzen durch Neuordnung zu andersartigen Substanzen. Fermentationsverfahren haben eine lange Tradition. Verfahren zur Herstellung von Brot, Wein, Bier, Käse, Quark usw. gibt es seit mehr als 6.000 Jahren.

Es gibt 5 bedeutende Gruppen der wirtschaftlich wichtigen Fermentation:
1. mikrobielle Zellen oder Biomasse als Produkt, z.B. einzelne Zellproteine, wie sie z.B. bei der Produktion von Bäckerhefe zum Einsatz kommt;
2. mikrobielle Enzyme: Katalase, Amylase, Protease, Pektinase, Glukoseisomerase, Zellulase, Hemizellulase, Lipase, Laktase, Streptokinase usw.;
3. mikrobielle Metaboliten:
   (a) primäre Metaboliten bzw. Stoffwechselprodukte, wie z.B. Ethanol, Zitronensäure, Glutaminsäure, Lysin, Vitamine, Polysaccharide usw.
   (b) sekundäre Metaboliten bzw. Stoffwechselprodukte: alle antibiotischen Fermentationsprodukte;
4. Rekombinationsprodukte, wie z.B. Insulin, HBV Hepatitis B Virus, Interferon, GCSF, Streptokinase;
5. Biotransformationsprodukte: Phenylacetylcarbinol, steroide Biotransformationsprodukte usw.

Wachstumsmedien (Substrate) sind für die industrielle Fermentation notwendig, da jede Mikrobe bzw. Mikroorganismus Wasser, Sauerstoff, eine Energiequelle, eine Kohlenstoffquelle, eine Stickstoffquelle sowie Mikronährstoffe für das Wachstum benötigt. Zucker aus Stärke, Zellulose, Zuckerrohr oder Zuckerrüben ist die häufigste gemeinsame Kohlenstoff- und Energiequelle für Fermentation. Typische Beispiele von Fermentationsprodukten sind Ethanol, Milchsäure und Wasserstoff. Allerdings können auch exotischere Verbindungen wie z.B. Buttersäure oder Aceton durch Fermentation hergestellt werden. Hefe vergärt im Rahmen der Herstellung von Bier, Wein oder anderen alkoholischen Getränken in einem Fermentationsprozess Zucker zu Ethanol, wobei große Mengen CO2 freigesetzt werden. Maltodextrine oder Laktose aus der Molke können ebenfalls als Kohlenstoffquelle dienen.

Manche Mikroorganismen sind auch dazu in der Lage, Fette von pflanzlichen Ölen oder Kohlenwasserstoffen von Petroleumfraktionen als Energiequelle zu metabolisieren.

Ein Fermentationsverfahren gemäß dem Oberbegriff des Anspruchs 1 ist aus der WO 2004/072291 A2 bekannt. Dieses Verfahren offenbart die Verwendung von Hopfensäuren in Fermentationsverfahren als Additiver gegen unerwünschte Mikroorganismen mit dem Ziel einer Ausbeuteerhöhung.

Die WO 2007/096673 A1 beschreibt ein Anti-Schaummittel, mit dem Ziel, hierdurch eine verbesserte Fermentationsrate zu erzielen. Als Anitschaummittel auf der Basis von Hopfenextrakt wird Hopfenöl vorgeschlagen.

Aus der WO 00/53814 ist ein Verfahren zur Bekämpfung von Mikroorganismen in einem zuckerhaltigen, wässrigen Prozessmedium bekannt. Auch hier dient der Einsatz der Hopfensäure zur Bekämpfung von im Zuckerproduktionsprozess von unerwünschten Mikroorganismen.

WO 00 52212 beschreibt ein Verfahren zur Kontrolle von Mikroorganismen in einem wässrigen Prozessmedium aus verdünnter Melasse bei der Herstellung von Hefe. Zur Vermeidung von unerwünschtem Bakterienwachstum in der Melasse bzw. im Melassesubstrat wird Hopfensäure zugegeben. Hopfensäure hat bakterizide Wirkung und ermöglicht es, mit Hilfe eines pflanzlichen Produktes das Bakterienwachstum bei der Fermentation von Hefe zu unterbinden.

WO 2007/096673 A1 beschreibt ein Verfahren, bei dem im Zuge eines Fermentationsprozesses Hopfenextrakt eingesetzt wird, um Schaumbildung zu verhindern.

Aus der DE OS 1 442 166 ist ein Verfahren zur Herstellung von Fruktose durch Fermentation bekannt, bei dem als Fermentationsbeschleuniger ein Sorbitmedium, das bestimmte Aminosäuren als Stickstoffquelle und bestimmte Schwermetalle enthält, vorgesehen ist.

Aus der DE AS 1 952 012 ist ein biotechnisches Verfahren zur Herstellung alkalischer Protease bekannt, bei dem dem Medium Hefeextrakt, Trockenhefe und Vitamine als Fermentationsbeschleuniger zugesetzt werden.

Eine Beschleunigung des Fermentationsprozesses kann auch durch verfahrenstechnische Maßnahmen erfolgen wie z.B. Einsatz einer besonderen Belüftung. Diese Maßnahmen erhöhen jedoch den verfahrenstechnischen Aufwand und die Investitionskosten beträchtlich.

### Aufgabe der Erfindung

Die Aufgabe der vorliegenden Erfindung besteht darin, einen verbesserten Fermentationsprozess mit erhöhter Beschleunigung und/oder erhöhter Ausbeute des Fermentationsprozesses zur Verfügung zu stellen.

### Prinzip der Erfindung

Die vorstehende Aufgabe wird durch den kennzeichnenden Teil des Anspruchs 1 gelöst. Die Unteransprüche betreffen zweckmäßige Ausgestaltungen der Erfindung.

Es hat sich überraschend herausgestellt, dass die vorgenannte Wirksubstanz zu einer beachtlichen Verbesserung des Fermentationsprozesses führt. Insbesondere kann die Ausbeute an dem Endprodukt oder dem zu erwartenden Zwischenprodukt wirksam erhöht werden. Vorteilhaft handelt es sich bei der zuvor beschriebenen Wirksubstanz um eine Wirksubstanz auf pflanzlicher Basis, also um keine Chemikalie. Obgleich Wirksubstanzen auf der Basis von Hopfen und/oder eines Derivats davon üblicherweise einen bitteren Geschmack haben, hat sich überraschend gezeigt, dass das Endprodukt oder zumindest Zwischenprodukt davon nicht nachteilig beeinträchtigt wird. Die erfindungsgemäße Wirksubstanz wirkt als Stimulator des Fermentationsprozesses.

Erfindungsgemäß handelt es sich bei der Wirksubstanz um Hopfenextrakt, insbesondere Hopfensäure, wie z.B. Alphasäure, Betasäure, Iso-Alphasäure, Rho-Iso-Alphasäure, Hexahydro-Iso-Alphasäure, Tetrahydro-Iso-Alphasäure, Hexahydro-Betasäure, oder einer solchen Säure oder einem Gemisch davon.

Auch sogenannte Hopfenöle und/oder Polyphenole aus dem Hopfen wie z. B. Xanthohumol können verwendet werden. Hopfenöl ist ein ätherisches Öl, welches zu 0,2 % bis 0,6 % im Hopfen enthalten ist. Das Hopfenöl wird aus Lupulin gebildet und gibt dem Hopfen das charakteristische Aroma.

Die Wirksubstanz ist erfindungsgemäß einzusetzen, unabhängig davon, ob eine Infektion des Wachstumsmediums (Substrat) mit unerwünschten (bzw. prozessfremden) Mikroorganismen oder Bakterien vorliegt oder nicht.

Zweckmäßigerweise ist das Fermentationshilfsmittel für den Einsatz in Substraten vorgesehen, die infektionsfrei oder nahezu infektionsfrei sind.

Das Fermentationshilfsmittel entfaltet seine die Fermentation stimulierende Wirkung unabhängig davon, ob es im Wachstumsmedium zu einer Schaumbildung kommt oder nicht.

Der Einsatz des Fermentationshilfsmittels führt zu einer erhöhten Ausbeute an Biomasse, Enzymen bzw. Metaboliten.

Das Fermentationshilfsmittel kann direkt dem Fermentationssubstrat, d.h. dem Wachstumsmedium, zugegeben werden.

Die Zugabe erfolgt gemäß einer zweckmäßigen Ausgestaltung kontinuierlich, so dass hierdurch bedingt eine konstant gehaltene Präsenz von Fermentationshilfsmittel in dem Wachstumsmedium vorliegt.

Alternativ kann je nach den Gegebenheiten auch eine diskontinuierliche Zugabe, vorzugsweise zu Beginn des jeweiligen Fermentationsprozesses, vorgenommen werden. So wird beispielsweise ein bestimmter Gehalt an Fermentationshilfsmittel in das Fermentationssubstrat eingebracht, wobei das Fermentationshilfsmittel in dem eingegebenen Umfang den anschließend ablaufenden Fermentationsprozess beschleunigend beeinflusst bzw. die Ausbeute erhöht.

Es wurde herausgefunden, dass bei Verwendung des erfindungsgemäßen Fermentationshilfsmittels zur Erzielung der die Fermentation beschleunigenden Wirkung bzw. der Ausbeuteerhöhung schon geringe Konzentrationen ausreichend sind, so dass der an sich bittere Geschmack des auf der Basis von Hopfen und/oder seinem Derivat beruhenden Wirkstoff keine den Geschmack negativ beeinträchtigende Wirkung entfaltet.

Zweckmäßigerweise liegt die Konzentration, insbesondere die sich einstellende Endkonzentration, des Fermentationshilfsmittels im Fermentationssubstrat in einem Bereich von 1 - 10000 ppm, insbesondere von 1 - 1000 ppm, vorzugsweise 1 - 500 ppm, besonders vorzugsweise 1 - 100 ppm.

### Beispiele

### Beispiel 1:

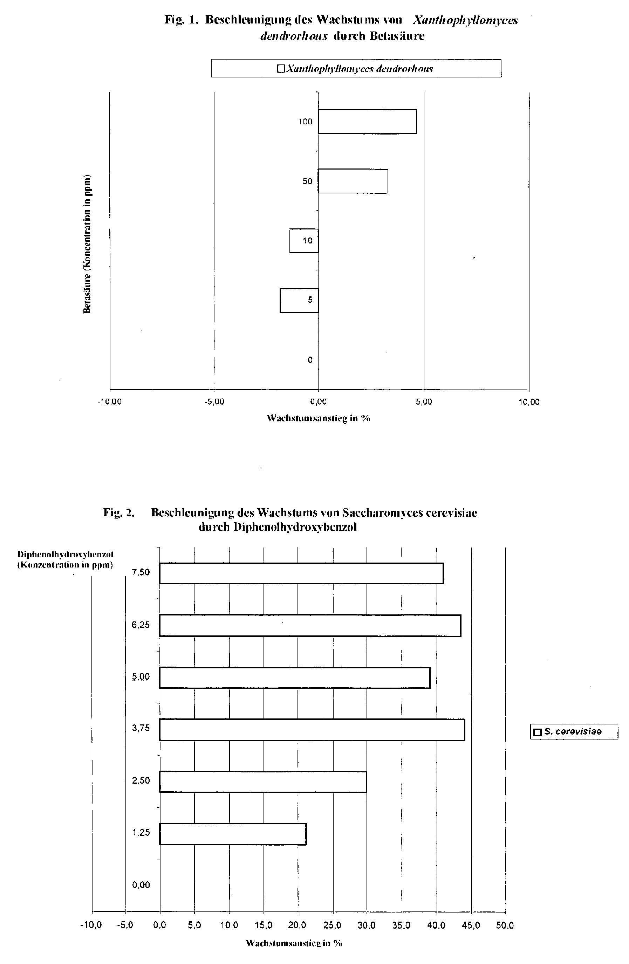

Der Mikroorganismus oder auch Schimmelpilz Xanthophyllomyces dendrohous produziert auf natürliche Weise eine Anzahl von Carotinoiden, die eine wirkungsvolle antioxidative Aktivität aufweisen. Untersuchungen haben gezeigt, dass das Wachstum von Xanthophyllomyces dendrohous im Beisein von Betasäuren signifikant erhöht wird (vgl. Fig. 1).

Der Mikroorganismus bzw. Schimmelpilz Xanthophyllomyces dendrohous wurde über Nacht in einem auf Zuckermolasse basierenden Wachstumsmedium bei einer Temperatur von 21,5 °C/200 rpm gezüchtet. Eine frische Kultur mit einem pH-Wert von 5,0 wurde mit 0, 5, 10, 50 bzw. 100 ppm Betasäure (aktive Substanz) versetzt und zu einer Über-Nacht-Kultur angesetzt (geimpft), um eine endgültige Zellkonzentration von 8 x 10⁵ KBE/ml zu erhalten. Die Kulturen wurden unter aeroben Bedingungen bei einer Temperatur von 21,5 °C/200 rpm für eine Dauer von ca. 24 Stunden bebrütet. Ein Kulturabsorptionsvermögen bei 600 nm wurde verwendet, um die Zelldichte zu bestimmen.

Die Untersuchung hat (wie Fig. 1 zeigt) folgendes Ergebnis ergeben:

In Konzentrationen ab 50 ppm tritt eine Erhöhung der Biomasseausbeute von Xanthophyllomyces dendrohous durch Betasäure auf.

## Patentansprüche

1. Fermentationsverfahren unter Verwendung eines Fermentationshilfsmittels, wobei letzteres als Beschleuniger der Fermentation und/oder zur Erhöhung der Ausbeute vorgesehen ist und eine Wirksubstanz auf pflanzlicher Basis umfasst,
**dadurch gekennzeichnet, dass**
es sich bei der Wirksubstanz auf pflanzlicher Basis um eine Wirksubstanz auf der Basis von Hopfen und/oder eines Derivats davon in Form von Alphasäure, Betasäure, Iso-Alphasäure, Rho-Iso-Alphasäure, Hexahydro-Iso-Alphasäure, Tetrahydro-Iso-Alphasäure, Hexahydro-Betasäure oder einem Gemisch davon oder
um ein Polyphenol aus dem Hopfen handelt und
das Fermentationshilfsmittel seine fermentationsbeschleunigende und/oder ausbeuteerhöhende Wirkung unabhängig von dem Vorliegen einer Infektion des Substrats an unerwünschten Mikroorganismen entfaltet.

2. Fermentationsverfahren nach Anspruch 1,
**dadurch gekennzeichnet, dass**
das Fermentationshilfsmittel seine fermentationsbeschleunigende und/oder ausbeuteerhöhende Wirkung unabhängig von dem Vorliegen einer Schaumbildung des Substrats entfaltet.

3. Fermentationsverfahren nach einem der vorhergehenden Ansprüche,
**dadurch gekennzeichnet, dass**
das Fermentationshilfsmittel für den Einsatz in Substraten vorgesehen ist, die frei von unerwünschten Mikroorganismen sind.

4. Fermentationsverfahren nach mindestens einem der vorhergehenden Ansprüche,
**dadurch gekennzeichnet, dass**
dieses als Beschleuniger der Fermentation und/oder zur Erhöhung der Ausbeute bei der Herstellung von Biomasse vorgesehen ist.

5. Fermentationsverfahren nach mindestens einem der vorhergehenden Ansprüche,
**dadurch gekennzeichnet, dass**
dieses als Beschleuniger der Fermentation und/oder zur Erhöhung der Ausbeute bei der Herstellung von Enzymen vorgesehen ist.

6. Fermentationsverfahren nach mindestens einem der vorhergehenden Ansprüche,
**dadurch gekennzeichnet, dass**
dieses als Beschleuniger der Fermentation und/oder zur Erhöhung der Ausbeute bei der Herstellung von Metaboliten oder Stoffwechselprodukten vorgesehen ist.

7. Fermentationsverfahren nach Anspruch 1,
**dadurch gekennzeichnet, dass**
das Fermentationshilfsmittel direkt zur Fermentation gegeben wird.

8. Fermentationsverfahren nach Anspruch 7,
**dadurch gekennzeichnet, dass**
die Zugabe des Fermentationshilfsmittels zur Fermentation kontinuierlich erfolgt.

9. Fermentationsverfahren nach Anspruch 7,
**dadurch gekennzeichnet, dass**
die Zugabe des Fermentationshilfsmittels zur Fermentation diskontinuierlich, insbesondere zu Beginn des Fermentationsprozesses, erfolgt.

10. Fermentationsverfahren nach den Ansprüchen 1 bis 9,
**dadurch gekennzeichnet, dass**
die Konzentration, insbesondere die sich einstellende Endkonzentration, des Fermentationshilfsmittels im Substrat in einem Bereich von 1 - 10000 ppm, liegt.

11. Verwendung einer Wirksubstanz auf der Basis von Hopfen und/oder eines Derivats davon in Form von
Alphasäure, Betasäure, Iso-Alphasäure, Rho-Iso-Alphasäure, Hexahydro-Iso-Alphasäure, Tetrahydro-Iso-Alphasäure, Hexahydro-Betasäure oder einem Gemisch davon oder
einem Polyphenol aus dem Hopfen
als Fermentationshilfsmittel zur Beschleunigung der Fermentation und/oder zur Erhöhung der Ausbeute , wobei
das Fermentationshilfsmittel seine fermentationsbeschleunigende und/oder ausbeuteerhöhende Wirkung unabhängig von dem Vorliegen einer Infektion des Substrats an unerwünschten Mikroorganismen entfaltet.

## Claims

1. Fermentation process using a fermentation auxiliary, wherein the latter is provided as an accelerator of the fermentation and/or for increasing the yield, and comprises a plant-based active ingredient,
**characterized in that**
the plant-based active ingredient is an active ingredient based on hops and/or a derivative thereof in the form of
alpha acid, beta acid, iso-alpha-acid, rho-iso-alpha-acid, hexahydro-iso-alpha-acid, tetrahydro-iso-alpha-acid, hexahydro-beta-acid or a mixture thereof or is a polyphenol from the hop and
the fermentation auxiliary develops its fermentation-accelerating and/or yield-increasing action independently of the presence of an infection of the substrate by unwanted microorganisms.

2. Fermentation process according to Claim 1,
**characterized in that**
the fermentation auxiliary develops its fermentation-accelerating and/or yield-increasing action independently of the presence of foam formation by the substrate.

3. Fermentation process according to any one of the preceding claims,
**characterized in that**
the fermentation auxiliary is provided for use in substrates which are free from unwanted microorganisms.

4. Fermentation process according to at least one of the preceding claims,
**characterized in that**
it is provided as accelerator of the fermentation and/or for increasing the yield in the production of biomass.

5. Fermentation process according to at least one of the preceding claims,
**characterized in that**
it is provided as accelerator of the fermentation and/or for increasing the yield in the production of enzymes.

6. Fermentation process according to at least one of the preceding claims,
**characterized in that**
it is provided as accelerator of the fermentation and/or for increasing the yield in the production of metabolites or metabolic products.

7. Fermentation process according to Claim 1,
**characterized in that**
the fermentation auxiliary is added directly to the fermentation.

8. Fermentation process according to Claim 7,
**characterized in that**
the fermentation auxiliary is added continuously to the fermentation.

9. Fermentation process according to Claim 7,
**characterized in that**
the fermentation auxiliary is added to the fermentation batchwise, in particular at the start of the fermentation process.

10. Fermentation process according to Claims 1 to 9,
**characterized in that**
the concentration, in particular the final concentration which is established, of the fermentation auxiliary in the substrate is in a range of 1-10 000 ppm.

11. Use of an active ingredient based on hops and/or a derivative thereof in the form of
alpha acid, beta acid, iso-alpha-acid, rho-iso-alpha-acid, hexahydro-iso-alpha-acid, tetrahydro-iso-alpha-acid, hexahydro-beta-acid or a mixture thereof or
a polyphenol from the hop as fermentation auxiliary for accelerating the fermentation and/or for increasing the yield, wherein
the fermentation auxiliary develops its fermentation-accelerating and/or yield-increasing action independently of the presence of an infection of the substrate by unwanted microorganisms.

## Revendications

1. Procédé de fermentation avec utilisation d'une auxiliaire de fermentation, ce dernier étant prévu en tant qu'accélérateur de la fermentation et/ou pour l'augmentation du rendement et comprenant une substance active à base végétale,
**caractérisé en ce que**
pour ce qui concerne la substance active à base végétale il s'agit d'une substance active à base de houblon et/ou d'un dérivé de celui-ci sous forme
d'acide alpha, acide bêta, acide iso-alpha, acide rho-iso-alpha, acide hexahydro-iso-alpha, acide tétrahydro-iso-alpha, acide hexahydro-bêta ou d'un mélange de ceux-ci ou
d'un polyphénol provenant du houblon et l'auxiliaire de fermentation déploie son action accélérant la fermentation et/ou augmentant le rendement indépendamment de la présence d'une infection du substrat par des micro-organismes indésirables.

2. Procédé de fermentation selon la revendication 1,
**caractérise en ce que**
l'auxiliaire de fermentation déploie son action accélérant la fermentation et/ou augmentant le rendement indépendamment de la présence d'une formation de mousse du substrat.

3. Procédé de fermentation selon l'une quelconque des revendications précédentes,
**caractérisé en ce que**
l'auxiliaire de fermentation est prévu pour l'utilisation dans des substrats qui sont exempts de micro-organismes indésirables.

4. Procédé de fermentation selon au moins l'une quelconque des revendications précédentes,
**caractérisé en ce que**
cette auxiliaire est prévu comme accélérateur de la fermentation et/ou pour l'augmentation du rendement dans la production de biomasse.

5. Procédé de fermentation selon au moins l'une quelconque des revendications précédentes,
**caractérisé en ce que**
cette auxiliaire est prévu comme accélérateur de la fermentation et/ou pour l'augmentation du rendement dans la production d'enzymes.

6. Procédé de fermentation selon au moins l'une quelconque des revendications précédentes,
**caractérisé en ce que**
cette auxiliaire est prévu comme accélérateur de la fermentation et/ou pour l'augmentation du rendement dans la production de métabolites ou de produits métaboliques.

7. Procédé de fermentation selon la revendication 1,
**caractérisé en ce que**
l'auxiliaire de fermentation est ajouté directement à la fermentation.

8. Procédé de fermentation selon la revendication 7,
**caractérisé en ce que**
l'addition de l'auxiliaire de fermentation à la fermentation s'effectue en continu.

9. Procédé de fermentation selon la revendication 7,
**caractérisé en ce que**
l'addition de l'auxiliaire de fermentation à la fermentation s'effectue en mode discontinu, en particulier au début du processus de fermentation.

10. Procédé de fermentation selon les revendications 1 à 9,
**caractérisé en ce que**
la concentration, en particulier la concentration finale qui s'établit, de l'auxiliaire de fermentation dans le substrat se situe dans une plage de 1 - 10 000 ppm.

11. Utilisation d'une substance active à base de houblon et/ou d'un dérivé de celui-ci sous forme d'acide alpha, acide bêta, acide iso-alpha, acide rho-iso-alpha, acide hexahydro-iso-alpha, acide tétrahydro-iso-alpha, acide hexahydro-bêta ou d'un mélange de ceux-ci ou
d'un polyphénol provenant du houblon comme auxiliaire de fermentation pour l'accélération de la fermentation et/ou pour l'augmentation du rendement,
l'auxiliaire de fermentation déployant son action accélérant la fermentation et/ou augmentant le rendement indépendamment de la présence d'une infection du substrat par des micro-organismes indésirables.
